# EUROPÄISCHE PATENTSCHRIFT

(11) **EP 0 625 134 B1**
(45) Veröffentlichungstag und Bekanntmachung des Hinweises auf die Patenterteilung: **19.07.1995**
(21) Anmeldenummer: 93909967.7
(22) Anmeldetag: 12.05.1993
(51) Int. Cl.: C07C 7/11

(54) **VERFAHREN ZUR GEWINNUNG LEICHTER C2+ -KOHLENWASSERSTOFFE AUS EINEM SPALTGAS**
METHOD OF PRODUCING LIGHT C2+ -HYDROCARBONS FROM CRACKED GAS
PROCEDE D'OBTENTION D'HYDROCARBURES LEGERS EN C2+ A PARTIR DE GAZ DE CRAQUAGE

(30) Priorität: 27.05.1992 DE 4217611
(43) Veröffentlichungstag der Anmeldung: 23.11.1994
(73) Patentinhaber: Linde Aktiengesellschaft, 65189 Wiesbaden (DE)
(72) Erfinder: BAUER, Heinz, D-81479 München (DE); BECKER, Hans, D-81479 München (DE)
(74) Vertreter: Kasseckert, Rainer
(86) Internationale Anmeldenummer: EP9301180
(87) Internationale Veröffentlichungsnummer: WO9324428

(56) Entgegenhaltungen:
- DE-B- 1 088 477
- US-A- 4 035 167

## Beschreibung

Die Erfindung betrifft ein Verfahren zur Gewinnung leichter C₂₊-Kohlenwasserstoffe, insbesondere Ethylen und Ethan, aus einem bei der Spaltung von Kohlenwasserstoffen gewonnenen und von schweren Kohlenwasserstoffen befreiten Gasgemisch.

Bei verschiedenen Spaltgasverfahren, vor allem beim Fluid-Catalytic-Cracking (FCC), fällt ein Gasgemisch an, das unter anderem leichte Kohlenwasserstoffe, insbesondere Ethylen und Ethan, aber auch C₃- und C₄-Kohlenwasserstoffe, enthält. In den meisten Raffinerien werden aus dem FCC-Abgas zunächst die schweren Kohlenwasserstoffe (C₅₊-Kohlenwasserstoffe) durch fraktionierte Destillation abgetrennt. Anschließend werden die C₃₊-Kohlenwasserstoffe durch Ölwäschen gewonnen, während die gesamten C₂-Kohlenwasserstoffe, aber auch ein gewisser Anteil an C₃₋- und C₄-Kohlenwasserstoffe ins Heizgasnetz abgegeben.

Sollen aus dem FCC-Abgas auch noch C₂₊-Kohlenwasserstoffe gewonnen werden, geschieht dies durch partielle Kondensation in einem Tieftemperaturprozeß. Ein derartiges Verfahren ist beispielsweise aus der EP-A-0 185 202 bekannt. Da die FCC-Abgase stets Spuren an höheren, mehrfach ungesättigten Kohlenwasserstoffen, Stickoxiden und Sauerstoff enthalten, besteht bei diesem Verfahren die Gefahr, daß sich in den tiefkalten Apparaten explosive Harze bilden. Eine Anlage mit partieller Kondensation in einem Tieftemperaturprozeß stellt damit immer ein gewisses Sicherheitsrisiko dar. So explodierte beispielsweise im Februar 1990 in Frankreich eine derartige Anlage nach achtjährigem Betrieb.

Aus der US-A-4 035 167 ist ein Verfahren zur Entfernung von Ethan und Ethylen aus einem Gasgemisch aus einer Kohlevergasung mittels Absorption bekannt, wobei als Waschmittel für die Absorption Cyclohexan, Cyclohexen, 1-Chlorobutan oder 1,1,1-Trichlorethan verwendet wird.

In der DE-B-1 088 477 wird ein Verfahren zur Absorption von Ethylen und Ethan aus einem Crackgas beschrieben, wobei nach Abtrennung von H₂, CO, N₂ und Methan das verbleibende C₂₊-Kohlenwasserstoffgemisch mit Verunreinigungen einer Acetonwäsche zugeführt wird.

Der vorliegenden Erfindung liegt daher die Aufgabe zugrunde, ein Verfahren der eingangs genannten Art aufzuzeigen, wobei die C₂₊-Kohlenwasserstoffe auf einfache Art und Weise aus dem Einsatzgas gewonnen werden können, aber die Sicherheit der Anlage durch die spezielle Verfahrensführung gewährleistet ist.

Diese Aufgabe wird erfindungsgemäß dadurch gelöst, daß die leichten C₂₊-Kohlenwasserstoffe aus dem Gasgemisch durch Absorption mit einem organischen, vorzugsweise paraffinischen, physikalisch wirkenden Waschmittel in einer Absorptionskolonne ausgewaschen werden, das ein Molekulargewicht zwischen 50 und 75 g/mol, vorzugsweise zwischen 60 und 75 g/mol, aufweist, wobei das Waschmittel in einem Kreislauf zunächst mit leichten Kohlenwasserstoffen beladen, in einer Regenerierkolonne regeneriert und anschließend wieder in die Absorptionskolonne zur erneuten Beladung geleitet wird.

Das erfindungsgemäße Verfahren hat den Vorteil, daß die leichten Kohlenwasserstoffe in der Gaswäsche aus dem Gasgemisch entfernt werden, wobei ein Sicherheitsrisiko vermieden wird, da die Bildung von explosiven Harzen bei dieser Verfahrensführung unterbunden wird.

Mit Vorteil wird als Waschmittel ein C₄₊-Kohlenwasserstoffschnitt, bevorzugt Pentan, Isopentan oder Mischungen daraus, eingesetzt. Ein derartiges Waschmittel zeichnet sich durch ein sehr gutes Lösungsvermögen für C₂-Kohlenwasserstoffe, insbesondere Ethylen und Ethan, aber auch für andere leicht löslichen Komponenten aus. Leichtbenzin, mit Anteilen im oben beschriebenen Molekulargewichtsbereich, insbesondere Pentanverbindungen, fallen bei der Vorbehandlung des Speisegases beispielsweise in der fraktionierten Destillation an und sind daher leicht verfügbar.

Bei einer bevorzugten Ausführungsform des erfindungsgemäßen Verfahrens wird das beladene Waschmittel vor der Einspeisung in die Regenerierkolonne mit einem Strippgas gestrippt, wobei insbesondere mitausgewaschenes Methan ausgestrippt wird. Das hierbei gewonnene Methan kann weiterverarbeitet werden oder beispielsweise als Heizgas verwendet werden.

Besondere Vorteile ergeben sich, wenn die Strippung des beladenen Waschmittels in einem unteren Abschnitt der Absorptionskolonne durchgeführt wird. Diese Verfahrensführung hat neben einem vereinfachten apparativen Aufwand den Vorteil, daß im Waschmittel gelöstes Methan auch zusätzlich zum Strippbereich aus dem Waschmittel bereits im Absorptionsbereich ausgestrippt werden kann, da das Strippgas die ganze Absorptionskolonne durchströmen kann.

Weitere Vorteile ergeben sich dadurch, daß als Strippgas verdampftes Waschmittel eingesetzt wird. Dazu wird im unteren Bereich der Absorptionskolonne Wärme, beispielsweise durch einen Aufkocher, zugeführt, so daß ein gewisser Teil des Waschmittels verdampft. Durch die tieferen Temperaturen im oberen Bereich der Absorptionskolonne kondensiert das verdampfte Waschmittel wieder aus und kann erneut mit leichten Kohlenwasserstoffen beladen werden. Ein Strippen mit einem externen Strippgas kann zu Verunreinigungen des Ethylenproduktstromes führen.

Das Gasgemisch wird mit Vorteil in die Absorptionskolonne mit einem Druck von 4 bis 50 bar, vorzugsweise 10 bis 30 bar, eingespeist. Ein erhöhter Druck bei der Absorption bedeutet einen höheren energetischen Aufwand, aber eine wesentliche Verringerung der erforderlichen Waschmittelmenge. So läßt sich der erforderliche Waschmittelfluß beispielsweise bei einer Druckerhöhung von 4 bar auf 30 bar um die Hälfte reduzieren.

Vorteilhafterweise wird die Absorption bei Temperaturen zwischen 0 und -50°C, vorzugsweise zwischen -30 und -50°C, besonders bevorzugt zwischen -35 und -45°C durchgeführt. Tiefere Temperaturen erhöhen das Lösevermögen des Waschmittels für die leichten Kohlenwasserstoffe, bedingen aber auf der anderen Seite eine mögliche Zunahme der NO₂-Bildung. Bei Einsatzströmen mit einem relativ hohen NOₓ-Gehalt sollte daher ein verhältnismäßig hohe Temperatur für die Absortion gewählt werden. Das kalte Überkopfprodukt der Absorptionskolonne kann zur Abkühlung des Spaltgasgemisches eingesetzt werden.

Nachdem die leichteren Komponenten als Ethylen, wie beispielsweise Methan, aus dem beladenen Waschmittel ausgestrippt wurden, wird das mit C₂₊-Kohlenwasserstoffen beladene Waschmittel in eine Regenerierkolonne geleitet. Die Regenerierung wird bei höheren Temperaturen als die Absorption durchgeführt. Die höchsten Temperaturen im erfindungsgemäßen Verfahren herrschen im unteren Bereich der Regeneriersäule. Dort wird, vorzugsweise durch einen Aufkocher, das Waschmittel erwärmt, um eine Desorption der C₃₋-Kohlenwasserstoffe aus dem Waschmittel zu erreichen. Mit Vorteil werden die Temperaturen im Sumpf der Regenerierkolonne dabei so gewählt, daß sie 120°C, vorzugsweise 105°C, nicht übersteigen. Es hat sich nämlich gezeigt, daß dann die Polymerisation, beispielsweise zu Butadien, verhindert wird. Polymerisate sind schwer aus dem Waschmittel zu entfernen. Die Temperatur im Sumpfbereich der Regenerierkolonne ist also nach unten hin durch eine ungenügende Desorption der C₃₋-Kohlenwasserstoffe aus dem Waschmittel und nach oben hin durch eine einsetzende Polymerisation gegeben.

Das Regenerieren des Waschmittels kann prinzipiell bei einem tieferen oder höheren Druck als die Absorption durchgeführt werden. Bevorzugt liegt der Druck in der Regeneriersäule aber unter dem der Absorptionskolonne.

Vom Kopf der Regeneriersäule wird ein Gasstrom abgezogen, teilweise kondensiert, in einen Abscheider geleitet und das Kondensat vom Abscheider zur Verringerung der Waschmittelverluste als Rückfluß wieder in die Regenerierkolonne eingespeist. Vom Abscheider wird außerdem ein C₂/C₃-Kohlenwasserstoff-reicher Produktgasstrom gewonnen. In die Regenerierung geleitete C₄₊-Kohlenwasserstoffe werden mit bzw. als regeneriertes Waschmittel vom Sumpf der Regeneriersäule zur Absorptionssäule zurückgeführt.

In Weiterbildung des erfindungsgemäßen Verfahrens wird das Kopfprodukt der Regenerierkolonne teilweise kondensiert, und nach Abtrennung und Rückführung des Kondensats in die Regenerierkolonne der C₂- und C₃-Kohlenwasserstoff-reiche Gasstrom einer C₂/C₃-Trennung zugeführt. In der C₂/C₃-Trennung wird ein C₂-Kohlenwasserstoff-Strom gewonnen, der in einer weiteren Trennstufe in Ethylen- und Ethan-haltige Produktgasströme aufgetrennt wird.

Die Erfindung wird im folgenden anhand eines Ausführungsbeispiels näher dargestellt.

Hierbei zeigt:

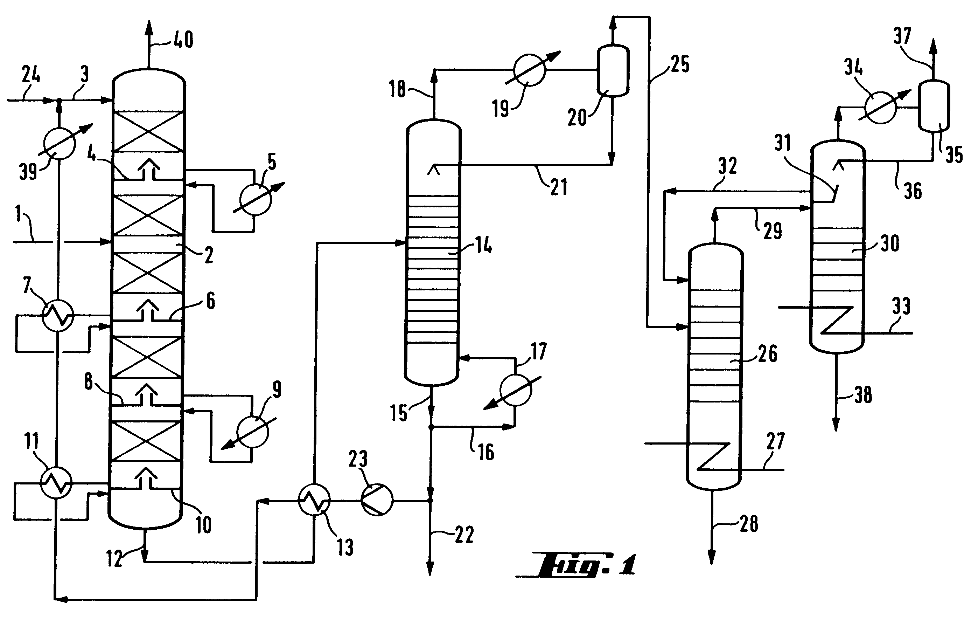

- Figur 1:: das erfindungsgemäße Verfahren mit Absorption, Regeneration und weiterer Auftrennung der C₂- und C₃-Kohlenwasserstoffe durch Destillation.

Anzumerken ist, daß die Summen der in den einzelnen Tabellen zur Zusammensetzung einzelner Ströme angeführten Werte aufgrund von Rundungen 100% übersteigen können.

Über Leitung 1 wird ein durch fraktionierte Destillation von C₄₊-Kohlenwasserstoffen befreites und getrocknetes FCC-Spaltabgas in die Absorptionskolonne 2 mit einem Druck von 13,3 bar und einer Temperatur von 22°C eingespeist. Das FCC-Abgas hat dabei folgende Zusammensetzung:

| | |
|---|---|
| H₂ | 3,4 Gew.-% |
| N₂ | 11,8 Gew.-% |
| CO | 1,1 Gew.-% |
| CH₄ | 31,6 Gew.-% |
| C₂H₄ | 20,5 Gew.-% |
| C₂H₆ | 28,4 Gew.-% |
| C₃H₆ | 2,0 Gew.-% |
| C₃H₈ | 1,0 Gew.-% |
| andere Komponenten | < 0,1 Gew.-% |

Über Leitung 3 wird in den oberen Bereich der Absorptionskolonne 2 regeneriertes Waschmittel, das zu etwa drei Vierteln aus Pentan und zu einem Viertel aus Isopentan besteht, mit einer Temperatur von -40°C und einem Druck von 13,1 bar eingespeist. Über dem Kaminboden 4 wird das Waschmittel abgezogen, im Seitenkühler 5 gekühlt, um die entstehende Prozeßwärme abzuführen, und wieder in die Absorptionskolonne eingeführt. Über dem Kaminboden 6 wird beladenes Waschmittel im indirekten Wärmetausch (7) mit regeneriertem Waschmittel angewärmt und wieder in die Absorptionskolonne 2 zurückgeführt. Über dem Kaminboden 8 wird das Waschmittel erneut abgezogen und im Aufkocher 9 weitererwärmt, bevor es wieder in die Absorptionskolonne 2 unterhalb des Kaminbodens 8 eingespeist wird. Über dem Kaminboden 10 wird das Waschmittel wiederum abgezogen und im indirekten Wärmetausch (11) mit regeneriertem Waschmittel weitererwärmt und in den untersten Abschnitt der Absorptionskolonne 2 eingespeist. Die drei Kolonnenabschnitte unterhalb des Kaminbodens 6 dienen der Entfernung von leichteren Komponenten als Ethylen aus dem Waschmittel. Durch die Erwärmung des Waschmittels entsteht Waschmitteldampf, der als Strippgas zum Ausstrippen der leichteren Komponenten als Ethylen, insbesondere Methan, eingesetzt wird. Über Kopf der Absorptionskolonne 2 wird daher ein nahezu C₂₊-Kohlenwasserstoff-freier Gasstrom mit folgender Zusammensetzung abgezogen:

| | |
|---|---|
| H₂ | 6,7 Gew.-% |
| N₂ | 23,0 Gew.-% |
| O₂ | 0,1 Gew.-% |
| CO | 2,2 Gew.-% |
| CH₄ | 61,6 Gew.-% |
| und Spuren von C₂₊-Kohlenwasserstoffen. | |

Vom Sumpf der Absorptionskolonne 2 wird über Leitung 12 ein mit C₂₊-Kohlenwasserstoffen beladenes Waschmittel mit einer Temperatur von 54°C und einem Druck von 13,4 bar abgezogen:

| | |
|---|---|
| C₂H₄ | 3,4 Gew.-% |
| C₂H₆ | 5,2 Gew.-% |
| C₃H₆ | 0,8 Gew.-% |
| C₃H₈ | 0,3 Gew.-% |
| C₅H₁₂ | 65,6 Gew.-% |
| C₅H₁₂(Iso) | 24,6 Gew.-% |
| andere Komponenten | < 0,1 Gew.-% |

Das beladene Waschmittel aus Leitung 12 wird im indirekten Wärmetausch (13) mit indirektem Waschmittel weitererwärmt und in die Regenerierkolonne 14 eingeleitet. In der Regenerierkolonne 14 findet die Warmregenerierung des Waschmittels statt. Dazu wird regeneriertes Waschmittel vom Sumpf der Regenerierkolonne (15) teilweise (16) im Aufkocher 17 auf 105°C erwärmt und in den unteren Bereich der Regenerierkolonne zurückgeführt. Der dabei entstehende Waschmitteldampf strippt die C₂- und C₃-Kohlenwasserstoffe aus dem Waschmittel aus. Das Überkopfprodukt der Regenerierkolonne (18) mit einer Temperatur von 57°C und einem Druck von 7,6 bar wird in einem Kondensator 19 gekühlt und einem Abscheider 20 zugeführt. Auskondensierte Bestandteile, zum überwiegenden Teil auskondensiertes Waschmittel, werden vom Abscheider 20 wieder in den oberen Bereich der Regenerierkolonne 14 zurückgegeben. Der vom Sumpf der Regenerierkolonne 14 über Leitung 15 mit einer Temperatur von 102°C und einem Druck von 7,8 bar abgezogene Waschmittelstrom, der nicht über Leitung 16 in die Regenerierkolonne zurückgeführt wird, wird durch Pumpe 23 auf einen Druck von 14 bar angehoben und in den Wärmetauscher 13, 11, 7 und 39 auf -40°C abgekühlt. Die Zusammensetzung des von der Regeneriersäule abgezogenen regenerierten Waschmittels lautet:

| | |
|---|---|
| C₂H₆ | 0,4 Gew.-% |
| C₃H₆ | 0,5 Gew.-% |
| C₃H₈ | 0,2 Gew.-% |
| C₅H₁₂ | 72,0 Gew.-% |
| C₅H₁₂(Iso) | 27,0 Gew.-% |
| andere Komponenten | < 0,1 Gew.-% |

Über Leitung 22 wird ein geringer Waschmittelpurge abgetrennt. Eine entsprechende Menge an Waschmittel wird über Leitung 24 dem abgekühlten Waschmittel in Leitung 3 zugegeben.

Vom Abscheider 20 wird ein C₂-/C₃-Kohlenwasserstoffgasgemisch in Leitung 15 mit folgender Zusammensetzung gewonnen:

| | |
|---|---|
| C₂H₄ | 38,7 Gew.-% |
| C₂H₆ | 55,2 Gew.-% |
| C₃H₆ | 3,7 Gew.-% |
| C₃H₈ | 1,9 Gew.-% |
| C₅H₁₂ | 0,2 Gew.-% |
| C₅H₁₂(Iso) | 0,4 Gew.-% |
| andere Komponenten | < 0,1 Gew.-% |

Dieser Gasstrom wird anschließend einer fraktionierten Destillation zugeführt und hierzu zunächst in Kolonne 26 für C₂-/C₃-Kohlenwasserstofftrennung eingeleitet. Kolonne 26 ist mit einer Sumpfheizung 27 ausgestattet. Vom Sumpf der Kolonne 26 wird ein im wesentlichen C₃-Kohlenwasserstoff-haltiges Destillat (LPG) über Leitung 28 mit folgender Zusammensetzung abgezogen:

| | |
|---|---|
| C₃H₆ | 58,8 Gew.-% |
| C₃H₈ | 30,9 Gew.-% |
| C₄H₆ | 0,3 Gew.-% |
| C₅H₁₂ | 3,2 Gew.-% |
| C₅H₁₂(Iso) | 6,7 Gew.-% |
| andere Komponenten | < 0,1 Gew.-% |

Das im wesentlichen C₂-Kohlenwasserstoff-haltige Überkopfprodukt mit der Zusammensetzung:

| | |
|---|---|
| C₂H₄ | 36,9 Gew.-% |
| C₂H₆ | 62,9 Gew.-% |
| C₃H₆ | 0,1 Gew.-% |
| andere Komponenten | < 0,1 Gew.-% |

wird über Leitung 29 in die Kolonne 30 eingespeist. Um die C₃H₆-/C₃H₈₊-Anteile in Leitung 29 möglichst gering zu halten, wird der oberhalb des Seitenbodens 31 abgezogene Strom 32 als Rückfluß in den oberen Bereich der Kolonne 26 zurückgeleitet. Der Rückfluß 32 setzt sich wie folgt zusammen:

| | |
|---|---|
| C₂H₄ | 24,6 Gew.-% |
| C₂H₆ | 75,3 Gew.-% |
| andere Komponenten | < 0,1 Gew.-% |

Kolonne 30 dient dem Aufsplitten der C₂-Verbindungen. Der Sumpfbereich von Kolonne 30 wird dazu über die Sumpfheizung 33 erwärmt, während das Überkopfprodukt von Kolonne 30 teilweise kondensiert (34), und das von einem Abscheider 35 abgezogene Kondensat als Rückfluß in Kolonne 30 über Leitung 36 zurückgeführt wird, um dort eine Rückwaschung der schwereren Komponenten als Ethylen zu erreichen. Das Überkopfprodukt der Kolonne 30 besteht zu über 99,9% aus Ethylen, so daß über Leitung 37 ein reiner Ethylenproduktstrom mit minimalen Verunreinigungen gewonnen wird. Über Leitung 38 wird vom Sumpf der Kolonne 30 ein Sumpfprodukt mit der Zusammensetzung:

| | |
|---|---|
| C₂H₄ | 0,9 Gew.-% |
| C₂H₆ | 98,8 Gew.-% |
| C₃H₆ | 0,3 Gew.-% |
| andere Komponenten | < 0,1 Gew.-% |

abgezogen. Diese Ethanfraktion aus Leitung 38 kann beispielsweise mit dem Überkopfprodukt der Absorptionskolonne 2 in Leitung 40 vermischt werden und als Heizgas verwendet werden.

Die Kolonnen 26 und 30 können in einer Kolonne kombiniert werden, wobei in diesem Fall vom Kopf dieser Kolonne ebenfalls ein Ethylenstrom gewonnen wird, während vom Sumpf der Kolonne ein C₃-Kohlenwasserstoff/Ethangemisch abgezogen wird.

## Patentansprüche

1. Verfahren zur Gewinnung leichter C₂₊-Kohlenwasserstoffe, insbesondere Ethylen und Ethan, aus einem bei der Spaltung von Kohlenwasserstoffen gewonnenen und von schweren Kohlenwasserstoffen befreiten Gasgemisch,
**dadurch gekennzeichnet,** daß die leichten C₂₊-Kohlenwasserstoffe aus dem Gasgemisch durch Absorption mit einem organischen, vorzugsweise paraffinischen, physikalisch wirkenden Waschmittel in einer Absorptionskolonne ausgewaschen werden, das ein Molekulargewicht zwischen 50 und 75 g/mol, vorzugsweise zwischen 60 und 75 g/mol, aufweist, wobei das Waschmittel in einem Kreislauf zunächst mit leichten Kohlenwasserstoffen beladen, in einer Regenerierkolonne regeneriert und anschließend wieder in die Absorptionskolonne zur erneuten Beladung geleitet wird.

2. Verfahren nach Anspruch 1, dadurch gekennzeichnet, daß als Waschmittel ein C₄₊-Kohlenwasserstoffschnitt, bevorzugt Pentan, Isopentan oder Mischungen daraus, eingesetzt werden.

3. Verfahren nach einem der Ansprüche 1 oder 2, dadurch gekennzeichnet, daß das beladene Waschmittel vor der Einspeisung in die Regenerierkolonne mit einem Strippgas gestrippt wird, wobei insbesondere mitausgewaschenes Methan ausgestrippt wird.

4. Verfahren nach Anspruch 3, dadurch gekennzeichnet, daß die Strippung des beladenen Waschmittels in einem unteren Abschnitt der Absorptionskolonne durchgeführt wird.

5. Verfahren nach Anspruch 3 oder 4, dadurch gekennzeichnet, daß als Strippgas verdampftes Waschmittel eingesetzt wird.

6. Verfahren nach einem der Ansprüche 1 bis 5, dadurch gekennzeichnet, daß das Spaltgas in die Absorptionskolonne mit einem Druck von 4 bis 50 bar, vorzugsweise 10 bis 30 bar, eingespeist wird.

7. Verfahren nach einem der Ansprüche 1 bis 6, dadurch gekennzeichnet, daß die Absorption bei Temperaturen zwischen 0 und -50°C, vorzugsweise zwischen -30 und -50°C, besonders bevorzugt zwischen -35 und -45°C, durchgeführt wird.

8. Verfahren nach einem der Ansprüche 1 bis 7, dadurch gekennzeichnet, daß die Temperatur im Sumpf der Regeneriersäule 120°C, vorzugsweise 105°C, nicht übersteigt.

9. Verfahren nach einem der Ansprüche 1 bis 8, dadurch gekennzeichnet, daß der Druck in der Regeneriersäule unter dem der Absorptionskolonne liegt.

10. Verfahren nach einem der Ansprüche 1 bis 9, dadurch gekennzeichnet, daß das vom Kopf der Regenerierkolonne abgezogene und nach seiner teilweisen Kondensation vom Kondensat abgetrennte, C₂-Kohlenwasserstoff- und C₃-Kohlenwasserstoff-haltige Gas einer C₂/C₃-Trennung zugeführt wird und daß von der C₂/C₃-Trennung abgezogene C₂-Kohlenwasserstoffe in Ethylen- und Ethan-haltige Produktgasströme aufgetrennt werden.

## Claims

1. Method of producing light C₂₊ hydrocarbons, in particular ethylene and ethane, from a gas mixture produced in the cracking of hydrocarbons and having been freed of high molecular weight hydrocarbons, characterized in that the low molecular weight C₂₊ hydrocarbons are scrubbed from the gas mixture by absorption by means of an organic, preferably paraffinic, physically acting scrubbing medium in an absorption column, the scrubbing medium having a molecular weight between 50 and 75 g/mol, preferably between 60 and 75 g/mol, the circulated scrubbing medium being first loaded with low molecular weight hydrocarbons, regenerated in a regeneration column and subsequently returned to the absorption column for renewed loading.

2. Method according to Claim 1, characterized in that a C₄₊ hydrocarbon fraction, preferably pentane, isopentane or mixtures thereof is employed as the scrubbing medium.

3. Method according to any one of Claims 1 or 2, characterized in that the loaded scrubbing medium prior to being fed into the regeneration column is stripped by means of a stripping gas, in particular co-absorbed methane being stripped off.

4. Method according to Claim 3, characterized in that the stripping of the loaded scrubbing medium is carried out in a lower section of the absorption column.

5. Method according to Claim 3 or 4, characterized in that vaporized scrubbing medium is employed as stripping gas.

6. Method according to any one of Claims 1 to 5, characterized in that the cracked gas is fed into the absorption column at a pressure of 4 to 50 bar, preferably 10 to 30 bar.

7. Method according to any one of Claims 1 to 6, characterized in that the absorption is carried out at temperatures between 0 and -50°C, preferably between -30 and -50°C, particularly preferably between -35 and -45°C.

8. Method according to any one of Claims 1 to 7, characterized in that the temperature in the bottom of the regeneration column does not exceed 120°C, preferably 105°C.

9. Method according to any one of Claims 1 to 8, characterized in that the pressure in the regeneration column is below that of the absorption column.

10. Method according to any one of Claims 1 to 9, characterized in that the gas containing C₂ hydrocarbons and C₃ hydrocarbons after having been withdrawn from the head of the regeneration column and separated from condensate after its partial condensation is subjected to a C₂/C₃ separation and in that C₂ hydrocarbons withdrawn from the C₂/C₃ separation are separated further into ethylene- and ethane-containing product gas streams.

## Revendications

1. Procédé de récupération d'hydrocarbures de poids moléculaire faible C₂₊, en particulier d'éthylène et d'éthane, à partir d'un mélange gazeux obtenu par craquage d'hydrocarbures et ayant été libéré des hydrocarbures de poids moléculaire élevé, caractérisé en ce que les hydrocarbures de poids moléculaire faible C₂₊ sont lavés du mélange gazeux par absorption au moyen d'un agent de lavage organique agissant physiquement, de préférence la paraffine, dans une colonne d'absorption, l'agent de lavage ayant un poids moléculaire compris entre 50 et 75 g par mole, de préférence entre 60 et 75 g par mole, l'agent de lavage étant passé dans un circuit pour être d'abord chargé d'hydrocarbures de poids moléculaire faible, étant régénéré dans une colonne de régénération et ensuite, étant retourné vers la colonne d'absorption pour être à nouveau chargé.

2. Procédé suivant la revendication 1, caractérisé en ce qu'une fraction d'hydrocarbures en C₄₊, de préférence, le pentane, l'isopentane ou des mélanges de ceux-ci, est utilisée comme agent de lavage.

3. Procédé suivant l'une quelconque des revendications 1 ou 2, caractérisé en ce que l'agent de lavage chargé, avant d'être amené vers la colonne de régénération, est rectifié par un agent de rectification, où on élimine, en particulier, le méthane coabsorbé.

4. Procédé suivant la revendication 3, caractérisé en ce que la rectification de l'agent de lavage chargé est réalisée dans une partie inférieure de la colonne d'absorption.

5. Procédé suivant la revendication 3 ou 4, caractérisé en ce que l'agent de lavage évaporé est utilisé comme gaz de rectification.

6. Procédé suivant l'une quelconque des revendications 1 à 5, caractérisé en ce que le gaz de craquage est amené vers la colonne d'absorption à une pression de 4 à 50 bars, de préférence, de 10 à 30 bars.

7. Procédé suivant l'une quelconque des revendications 1 à 6, caractérisé en ce que l'absorption est réalisée à des températures entre 0 et -50°C, de préférence entre -30 et -50°C, en particulier entre -35 et -45°C.

8. Procédé suivant l'une quelconque des revendications 1 à 7, caractérisé en ce que la température dans la cuve de la colonne de régénération n'excède pas 120°C, de préférence 105°C.

9. Procédé suivant l'une quelconque des revendications 1 à 8, caractérisé en ce que la pression dans la colonne de régénération est inférieure à celle de la colonne d'absorption.

10. Procédé suivant l'une quelconque des revendications 1 à 9, caractérisé en ce que le gaz contenant des hydrocarbures en C₂ et des hydrocarbures en C₃, après avoir été extrait de la tête de la colonne de régénération et être séparé du condensat après sa condensation partielle, est soumis à une séparation C₂/C₃ et les hydrocarbures en C₂ extraits de la séparation C₂/C₃ sont séparés ensuite en courants gazeux contenant de l'éthylène et contenant de l'éthane.
